# DEMANDE DE BREVET EUROPEEN

(11) **EP 4 585 259 A2**
(43) Date de publication de la demande: **16.07.2025**
(21) Numéro de dépôt: 25180050.4
(22) Date de dépôt: 04.06.2024
(51) Int. Cl.: A61N 5/06

(54) **DISPOSITIF DE TRAITEMENT PAR PHOTOTHÉRAPIE**

(30) Priorité: 07.06.2023 FR 2305756
(62) Demande divisionnaire de: 24180051.5
(71) Demandeur: Lucibel SA, 76770 Le Houlme (FR)
(72) Inventeur: DAVID, Thibault, 76240 Bonsecours (FR); GONTIER, Paul, 76100 Rouen (FR)
(74) Mandataire: Martin, Marie-Aude

(57) **Abrégé**

Le dispositif (10) de traitement du cuir chevelu d'un utilisateur par photothérapie, comprenant un corps comprenant des parois extérieure (22) et intérieure (24) et une source de lumière (16) disposée entre les deux parois (22, 24) émettant d'un côté de la paroi intérieure (24) du corps. Le corps présente un motif (50) qui apparaît lors de l'allumage du dispositif (10) par effet de contraste de translucidité sur fond opaque ou d'opacité sur fond translucide.

## Description

La présente invention concerne un dispositif de traitement par photothérapie pour une région externe du corps d'un être vivant. Plus particulièrement mais non exclusivement, elle s'applique au traitement par photothérapie de la surface de tête d'un utilisateur, par exemple au traitement du cuir chevelu en tant que traitement de la calvitie.

De façon connue en soi, le traitement consiste en l'application d'un rayonnement lumineux présentant un spectre d'émission prédéfini directement sur le cuir chevelu. Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande relativement étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du métabolisme cellulaire. Une telle régénération cellulaire va se manifester par exemple pour les cellules de la peau du visage par une augmentation de la production de collagène et d'élastine et par conséquent une diminution observable des rides et ridules.

Plus particulièrement, il a été observé que l'exposition du cuir chevelu à la lumière rouge, par exemple à la longueur d'onde de 630 nm, permet une stimulation de la repousse capillaire, une amélioration de la qualité et de la densité des cheveux ainsi qu'une stabilisation de la perte de cheveux.

Les objets de photothérapie sont désormais considérés comme des produits de bien-être et doivent s'intégrer tant dans le quotidien que dans l'environnement des utilisateurs. Ces objets doivent donc être à la fois ergonomiques mais également esthétiques en offrant un aspect visuel global de qualité premium.

Différentes caractéristiques permettent d'apporter une perception de qualité telles que le choix des matériaux, le poids, l'ergonomie.

L'invention propose une solution alternative conférant à l'objet de photothérapie une perception de qualité et de technicité accrue et originale.

### Description de l'invention

A cet effet, l'invention a pour objet un dispositif de traitement par photothérapie selon la revendication principale.

Un dispositif selon l'invention peut comprendre l'une ou plusieurs des caractéristiques suivantes.

Dans un mode de réalisation préféré de l'invention, le dispositif comprend une couche de dissipation thermique réalisée dans un matériau opaque à la lumière qui s'étend entre les deux parois, la couche étant localement évidée selon ledit motif.

Dans un mode de réalisation préféré de l'invention, la paroi extérieure étant réalisée dans un matériau translucide, la paroi extérieure présente localement une surépaisseur suivant le motif qui empêche la lumière de passer au travers.

Dans un mode de réalisation préféré de l'invention, le corps présente une concavité orientée vers la paroi intérieure, la paroi extérieure comprend un bord distal et le motif est formé par une surépaisseur périphérique le long du bord distal à distance de l'extrémité libre du bord distal.

Dans un mode de réalisation préféré de l'invention, la paroi extérieure s'évase vers l'extérieur pour former une bordure décorative circonférentielle le long du bord distal délimitée par un décrochement de paroi qui forme la surépaisseur périphérique.

Dans un mode de réalisation préféré de l'invention, la paroi extérieure étant réalisée dans un matériau opaque, la paroi extérieure présente localement un amincissement suivant le motif qui laisse la lumière passer au travers.

Dans un mode de réalisation préféré de l'invention, le corps a une forme de casque pour recouvrir le cuir chevelu d'un utilisateur.

Dans un mode de réalisation préféré de l'invention, le casque définit une fois les parois extérieure et intérieure assemblées, une bordure périphérique qui délimite une ouverture de passage de la tête de l'utilisateur et une source de lumière disposée entre les deux parois émettant vers l'intérieur du casque.

Dans un mode de réalisation préféré de l'invention, un bandeau circonférentiel d'ajustement du casque à un tour de tête de l'utilisateur, accroché à la bordure périphérique, ledit bandeau comprenant un corps pourvu d'une portion de déformation s'étendant radialement vers l'intérieur du casque et étant configurée pour se déformer entre une position de repos déployée radialement vers l'intérieur du casque et une position tassée entre le casque et la tête de l'utilisateur en configuration d'utilisation.

Dans un mode de réalisation préféré de l'invention, le corps a une forme de masque facial.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La figure 1 est une vue en perspective d'un dispositif de photothérapie selon un premier mode de réalisation comprenant un casque de traitement du cuir chevelu ;
[Fig. 2] La figure 2 est une vue de dessous du casque de la figure 1 ;
[Fig. 3] La figure 3 est une vue en coupe du casque de la figure 1.

### Description détaillée de l'invention

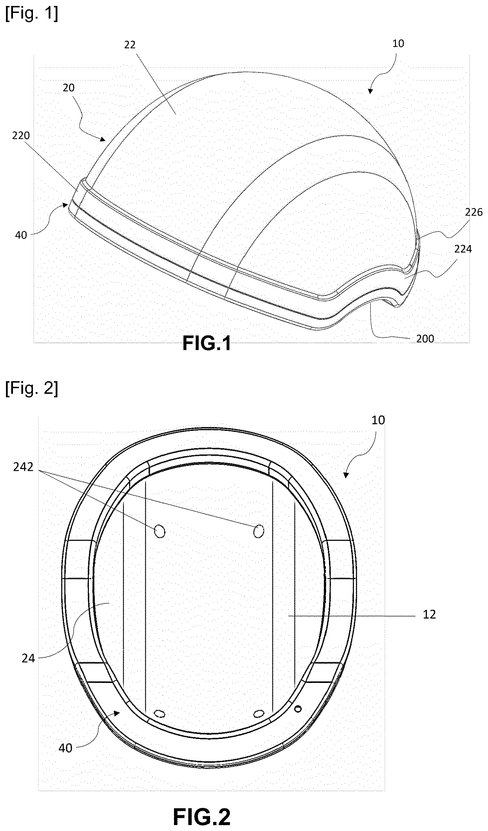

On a représenté sur les figures 1 à 3 un dispositif de photothérapie selon un premier mode de réalisation l'invention. Le dispositif est désigné par la référence générale 10 et comprend un casque de traitement du cuir chevelu selon un premier mode de réalisation de l'invention illustré par les figures 1 à 3.

Comme illustré sur les figures 1 à 3, le casque de traitement 10 comprend un corps 20 qui délimite une cavité 12 à l'intérieur de laquelle un utilisateur peut insérer sa tête. Le corps 20 incorpore encore une source de lumière 16 qui émet vers l'intérieur de la cavité 12 en direction de la surface à traiter. La source de lumière 16 comprend dans cet exemple une pluralité de diodes électroluminescentes. Dans l'exemple décrit, les diodes électroluminescentes émettent une lumière rouge de préférence avec une longueur d'onde autour de 630 nanomètres, dans une plage de 620 à 640 nanomètres. En variante, d'autres longueurs d'onde de lumière peuvent être sélectionnées en fonction de la nature du traitement de photothérapie souhaité.

Comme cela est illustré sur les figures, le corps 20 du casque 10 comprend des parois de coque extérieure 22 et intérieure 24 s'étendant chacune d'une portion de sommet proximale jusqu'à un bord distal de chaque paroi de coque 22 et 24. Les deux parois de coque 22 et 24, définissent une fois assemblées, une bordure périphérique 200 du casque 10 qui délimite l'ouverture de passage de la tête.

La paroi de coque intérieure 24 est réalisée dans une matière laissant passer le flux lumineux émis par la source de lumière 16. De préférence, la matière est translucide pour permettre une diffusion homogène de la lumière ainsi qu'une protection naturelle de l'utilisateur contre l'éblouissement. Par translucide, on entend un matériau qui laisse passer la lumière mais qui n'est pas nécessairement transparent. Le choix d'un matériau translucide plutôt qu'un matériau transparent permet également au niveau esthétique de rendre moins visibles les points lumineux formés par les diodes électroluminescentes. Bien entendu, en variante, le matériau peut être choisi transparent aux rayons lumineux émis par les sources lumineuses.

Par exemple, de préférence, la paroi intérieure 24 est réalisée dans une matière plastique thermoformable, par exemple un mélange de polystyrène et de polyéthylène (acronyme PS-PE).

Dans le mode de réalisation préféré de l'invention, la source de lumière 16 est disposée entre les deux parois de coque 22, 24 et émet vers l'intérieur du casque 10.

En outre, de préférence, les deux parois 22 et 24 sont accouplées entre elles par des attaches 244, par exemple en matière plastique se présentant sous forme de clips par enclenchement réciproque. En outre, les deux parois 22, 24 comprennent des orifices 242 coïncidant des deux parois 22 et 24. De préférence, les deux parois de coque 22 et 24 sont alors assemblées l'une avec l'autre par une pluralité d'éléments de liaison permanents ou réversibles insérés à l'intérieur des orifices 242.

Dans le mode de réalisation préféré de l'invention, le casque 10 comprend encore un bandeau circonférentiel 40 d'ajustement du casque 10 à un tour de tête de l'utilisateur, accroché à la bordure périphérique 200. Ce bandeau 40 permet d'ajuster la cavité 12 du casque 10 au tour de tête de l'utilisateur, ce tour de tête présentant une grande variabilité au sein de la population générale.

En particulier, ce bandeau 40 comprend un corps pourvu d'une portion de déformation 46 s'étendant radialement vers l'intérieur du casque 10 qui est configurée pour se déformer entre une position de repos déployée radialement vers l'intérieur du casque 10 et une position tassée entre le casque et la tête de l'utilisateur en configuration d'utilisation. De préférence, cette portion de déformation 46 est creuse ce qui permet de guider la déformation selon une ligne de déformation préférentielle. En outre, le bandeau 40 comprend deux portions de rattachement 48 et 44 respectivement par l'extérieur de la paroi externe 22 et par l'intérieur de la paroi interne 24.

De préférence, le bandeau 40 est réalisé dans une matière comprenant un élastomère de polyuréthane ou de silicone. Le matériau présente par exemple une dureté Shore A comprise entre 25 et 95 shore. Dans l'exemple illustré, le bandeau 40 est réalisé dans un matériau translucide

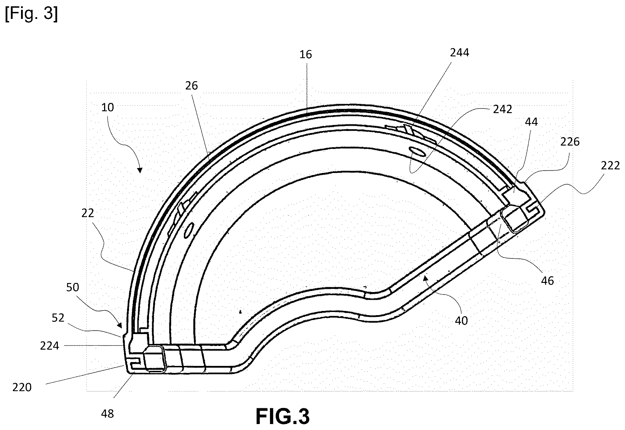

En outre, comme illustré sur la figure 3, le casque 10 comprend encore une couche de dissipation thermique 26, par exemple réalisée dans une plaque métallique de préférence en aluminium. De préférence, la plaque 26 est conformée pour s'étendre entre les deux parois 22 et 24 de coque du casque 10. Par exemple, l'interface de dissipation thermique 26 est formée par une plaque métallique conformée pour épouser la forme hémisphérique de la cavité 12 du corps de casque 10. De préférence, la plaque 26 est obtenue par pliage d'un flan métallique prédécoupé ou entaillé selon des lignes de pliage et/ou déformation préférentielles.

De préférence, le bandeau d'ajustement 40 du casque 10 à la tête de l'utilisateur est réalisé dans un matériau translucide à la lumière.

Dans le mode de réalisation préféré de l'invention, le corps 20 présente un motif 50 qui apparaît lors de l'allumage du dispositif 10 par effet de contraste de translucidité sur fond opaque ou d'opacité sur fond translucide.

Dans le mode de réalisation illustré sur les figures 1 à 3, la paroi de coque extérieure 22 étant réalisée dans un matériau translucide, la paroi de coque extérieure 22 présente localement une surépaisseur 52 qui empêche la lumière de passer délimitant alors le motif 50.

De préférence, la couche de dissipation thermique 26 est réalisée dans un matériau opaque à la lumière de sorte que la lumière n'est globalement pas émise vers l'extérieur du casque 10 dans toute une surface du casque hémisphérique couverte par la couche 26. Comme cela est visible sur la figure, la couche 26 ne s'étend pas jusqu'au bord d'extrémité 222 de la paroi de coque extérieure 22 de sorte qu'une bordure distale 220 de la paroi de coque extérieure 22 est traversée par la lumière.

Par exemple, le bord distal 220 de la paroi de coque extérieure 22 comprend une surépaisseur périphérique 52 ou bourrelet le long du bord distal 220, à distance de l'extrémité libre 222 du bord distal 220 qui forme une zone opaque à la lumière définissant le motif 50. Cette zone a le motif 50 en forme de liseré périphérique qui apporte un style particulier au casque 10 lors de l'utilisation.

De préférence, comme illustré sur la figure 3, la paroi extérieure 22 s'évase vers l'extérieur pour former une collerette décorative circonférentielle 224 le long du bord distal 220, formée en relief par rapport à la surface extérieure du casque 10. Par exemple, la collerette 224 est délimitée par un décrochement de la paroi externe 22, ce décrochement formant un bourrelet de matière épaissie au travers duquel la lumière ne peut pas passer.

Ainsi, lors de l'allumage de la source de lumière, le bord distal comprenant la collerette s'éclaire et le liseré périphérique apparaît par effet de contraste d'opacité sur fond translucide.

Dans une première variante non illustrée, la couche ou interface de dissipation thermique 26 est réalisée dans un matériau opaque à la lumière qui s'étend entre les deux parois de coque 22 et 24, la couche 26 étant localement évidée selon ledit motif.

Dans une deuxième variante non illustrée, la paroi extérieure 22 étant réalisée dans un matériau opaque, la paroi extérieure 22 présente localement un amincissement suivant le motif qui laisse la lumière passer au travers.

Selon un deuxième mode de réalisation de l'invention non illustré sur les figures, le dispositif de photothérapie peut comprendre un masque facial de photothérapie. Le masque comprend de préférence un corps formé par deux parois de coque entre lesquelles est intercalée une source lumineuse et une couche de dissipation thermique opaque. La couche de dissipation thermique opaque est évidée localement selon un motif qui apparaît par effet de contraste sur un fond opaque lors de l'allumage de la source de lumière.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Dispositif (10) de traitement par photothérapie pour une région du corps d'un utilisateur, comprenant un corps (20) en forme de casque pour recouvrir le cuir chevelu de l'utilisateur ou de masque facial, ledit corps comprenant des parois extérieure (22) et intérieure (24) et une source de lumière (16) disposée entre les deux parois (22, 24) émettant d'un côté de la paroi intérieure du corps, **caractérisé en ce que** le corps (20) présente un motif (50) qui apparaît lors de l'allumage du dispositif (10) par effet de contraste de translucidité sur fond opaque ou d'opacité sur fond translucide et **en ce que** le dispositif comprend une couche (26) de dissipation thermique réalisée dans un matériau opaque à la lumière qui s'étend entre les deux parois (22, 24), la couche (26) étant localement évidée selon ledit motif (50).

2. Dispositif (10) selon la revendication précédente, dans lequel la paroi extérieure (22) étant réalisée dans un matériau translucide, la paroi extérieure (22) présente localement une surépaisseur (226) suivant le motif (50) qui empêche la lumière de passer au travers.

3. Dispositif (10) selon la revendication précédente, dans lequel le corps (20) présente une concavité orientée vers la paroi intérieure (24), la paroi extérieure (22) comprend un bord distal (220) et le motif (50) est formé par une surépaisseur périphérique (52) le long du bord distal (220) à distance de l'extrémité libre (222) du bord distal (220).

4. Dispositif (10) selon la revendication précédente, dans lequel la paroi extérieure (22) s'évase vers l'extérieur pour former une collerette décorative circonférentielle (224) le long du bord distal (220) délimitée par un décrochement de paroi qui forme la surépaisseur périphérique (52).

5. Dispositif (10) selon la revendication 1, dans lequel la paroi extérieure (22) étant réalisée dans un matériau opaque, la paroi extérieure (22) présente localement un amincissement suivant le motif (50) qui laisse la lumière passer au travers.

6. Dispositif (10) selon l'une quelconque des revendications précédentes, dans lequel, **le corps ayant une forme de casque,** le casque (10) définit, une fois les parois extérieure (22) et intérieure (24) assemblées, une bordure périphérique (200) qui délimite une ouverture de passage de la tête de l'utilisateur et une source de lumière disposée entre les deux parois (22, 24) émettant vers l'intérieur du casque (10).

7. Dispositif (10) selon la revendication précédente, dans lequel un bandeau circonférentiel (40) d'ajustement du casque (10) à un tour de tête de l'utilisateur, accroché à la bordure périphérique (200), ledit bandeau (40) comprenant un corps pourvu d'une portion de déformation (46) s'étendant radialement vers l'intérieur du casque (10) et étant configurée pour se déformer entre une position de repos déployée radialement vers l'intérieur du casque (10) et une position tassée entre le casque (10) et la tête de l'utilisateur en configuration d'utilisation.
